# EUROPEAN PATENT SPECIFICATION

(11) **EP 1 805 127 B1**
(45) Date of publication and mention of the grant of the patent: **26.11.2014**
(21) Application number: 05804240.9
(22) Date of filing: 12.10.2005
(51) Int. Cl.: C07C 51/47, C07C 51/43, C07C 63/26, C07C 63/15, C07C 63/04

(54) **PROCESS FOR REMOVAL OF IMPURITIES FROM AN OXIDIZER PURGE STREAM**
VERFAHREN ZUM ENTFERNEN VON UNREINHEITEN AUS EINEM OXIDATIONSMITTELSÄUBERUNGSSTROM
PROCÉDÉ D'ÉLIMINATION D'IMPURETÉS D'UN COURANT DE PURGE OXYDANT

(30) Priority: 28.10.2004 US 975256; 28.10.2004 US 975252
(43) Date of publication of application: 11.07.2007
(73) Proprietor: GRUPO PETROTEMEX, S.A. DE C.V., San Pedro Garza Garcia, Nuevo Leon 66265 (MX)
(72) Inventor: GIBSON, Philip, Edward, Kingsport, TN 37660 (US); PARKER, Kenny, Randolph, Afton, TN 37616 (US); JENKINS, Howard, Wood, Jr., Pendleton, SC 29670 (US)
(74) Representative: Tostmann, Holger Carl
(86) International application number: PCT/US2005/036541
(87) International publication number: WO 2006/049818

(56) References cited:
- US-A- 2 964 559
- US-A- 3 950 409
- US-A- 4 356 319

## Description

### FIELD OF INVENTION

This invention relates to the removal of impurities and the recovery of a mother liquor and a wash filtrate from an oxidizer purge stream produced in the synthesis of carboxylic acid, typically terephthalic acid, and then subsequently routing at least a portion of the mother liquor and/or wash filtrate back to an oxidation zone comprising at least one oxidation reactor.

### BACKGROUND OF THE INVENTION

Terephthalic acid is commercially produced by oxidation of paraxylene in the presence of a catalyst, such as, for example, Co, Mn, Br and a solvent. Terephthalic acid is used in the production of polyester fibers, films, and resins and must be further treated to remove impurities formed as a result of the oxidation of paraxylene.

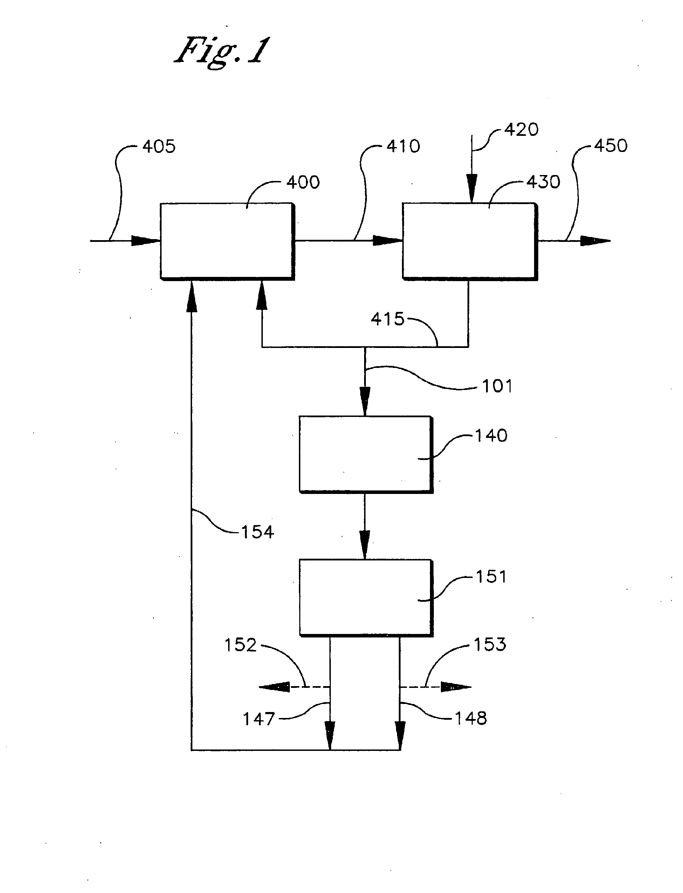

Terephthalic acid (TPA) is an intermediate in the production of polyesters for plastics and fiber applications. An example of a process for manufacturing TPA is shown in figure 1. Commercial processes for the manufacture of TPA are often based on the heavy-metal catalyzed oxidation of p-xylene (see e.g. U.S. #3,950,409), generally with a bromide promoter in an acetic acid solvent or the combinations of these can be known as the reaction mixture **405.** Due to the limited solubility of TPA in acetic acid under practical oxidation conditions, a slurry of TPA crystals is usually formed in the oxidation zone **400** which comprises at least one oxidation reactor. Typically, the TPA oxidizer slurry **410** is withdrawn from the oxidation zone **400,** and TPA solids can then be separated from the oxidizer mother liquor **415** in a TPA solid liquid separation zone **430,** using conventional solid- liquid separation techniques. The oxidizer mother liquor **415,** which contains most of the catalyst and promoter used in the process, is recycled to the oxidation zone **400** comprising at least one reactor. An appropriate process for recovering heavy metal oxidation catalysts is e.g. disclosed in U.S. #2,964,559, whereas U.S. #4,356,319 discloses a method of recovering an active form catalyst used in the synthesis of terephtalic acid. Further WO #2001/12318 discloses an invention relating to a process for the recovery and recycle of the oxidation catalyst. Aside from the catalyst and promoter, the oxidizer mother liquor **415** also contains dissolved TPA and many by-products and impurities. These by-products and impurities arise partially from minor impurities present in the p-xylene feed stream. Other impurities arise due to the incomplete oxidation of p-xylene resulting in partially oxidized products. Still other by-products result from competing side reactions formed as a result of the oxidation of p-xylene to terephthalic acid. Patents disclosing the production of terephthalic acid such as U. S patent #4,158,738 and #3,996,271 are established.

The TPA solids can undergo a solid-liquid separation wherein fresh solvent **420** is utilized to displace a major portion of the liquid component of the oxidizer mother liquor **415.** After drying, the TPA solids are contaminated with impurities that were present in the oxidizer mother liquor **415** since these impurities may be incorporated into the TPA solids. Impurities are also present due to occlusions in the TPA crystal structure and due to incomplete removal of the oxidizer mother liquor **415** by the fresh solvent wash **420.**

Many of the impurities in the oxidizer mother liquor **415** stream that is recycled are relatively inert to further oxidation. Such impurities include, for example, isophthalic acid, phthalic acid and trimellitic acid. Impurities, which may undergo further oxidation are also present, such as, for example, 4- carboxybenzaldehyde, p-toluic acid and p-tolualdehyde. Oxidation inert impurities tend to accumulate in the oxidizer mother liquor **415** upon recycle. The concentration of these inert impurities will increase in the oxidizer mother liquor **415** until an equilibrium is reached whereby the rate of removal of each impurity via the TPA product balances with the rate of formation and the rate of addition to the oxidation process. The normal level of impurities in commercial terephthalic acid makes it unsuitable for direct use in most polymer applications.

Conventionally, terephthalic acid has been purified either by conversion to a dimethyl ester or by dissolution in water with subsequent hydrogenation over standard hydrogenation catalysts. More recently, secondary oxidative treatments have been used to produce polymer-grade TPA. It is desirable to minimize the concentration of impurities in the mother liquor and thereby facilitate subsequent purification of TPA. In some cases, it is not possible to produce a purified, polymer-grade TPA unless some means for removing impurities from the oxidizer mother liquor **415** is utilized.

One technique for impurity removal from a recycle stream commonly used in the chemical processing industry is to draw out or "purge" some portion of the oxidizer mother liquor **415** that is recycled. Typically, the purge stream is simply disposed of or, if economically justified, subjected to various treatments to remove undesired impurities while recovering valuable components. One example is U.S. # 4,939,297. The amount of purge required for control of impurities is process-dependent; however, a purge amount equal to 10- 40 %, hereafter known as oxidizer purge stream **101**, of the total oxidizer mother liquor **415** stream that is recycled is usually sufficient to produce TPA adequate as feedstock for commercial polymer manufacture. However, in an embodiment of the invention a purge amount up to 100 % could be used.

In the production of TPA, the percentage purge of the oxidizer mother liquor **415** necessary to maintain acceptable impurity concentrations, coupled with the economic value of the metal catalyst and solvent components in the oxidizer purge stream **101,** make simple disposal of the oxidizer purge stream **101** economically unattractive. Thus, there is a need for a process that recovers a major portion of the valuable metal catalysts and acetic acid contained in the oxidizer purge stream **101** while removing a major portion of the impurities present in the oxidizer purge stream **101.** The metal catalyst can be recovered in an active form suitable for reuse by direct recycling to the p-xylene oxidation step.

Disclosed is an invention to recover a wash filtrate **148** and mother liquor **147** from an oxidizer purge stream. The oxidizer purge stream **101** is sent to a separation zone and then the mother liquor/and or wash filtrate is recycled back to an oxidation reactor. In should be noted that the above described TPA process is for example only. The disclosed invention can apply to many different TPA processes that are capable of producing an oxidizer purge stream **101.** Therefore, it should be noted that this invention does not just apply to the terephthalic acid process, but any process that produces an oxidizer purge stream **101** where recovery of a mother liquor **147** and wash filtrate **148** comprising a metal catalyst is needed.

### SUMMARY OF THE INVENTION

This invention relates to removal of impurities and the recovery of at least a portion of a mother liquor and wash filtrate from an oxidizer purge stream produced in the synthesis of carboxylic acids, typically terephthalic acid.

It is an object of this invention to provide a process to remove impurities and to recover a mother liquor and wash filtrate from an oxidizer purge stream.

It is yet another object of this invention to provide a process for the removal of impurities and the recovery of a mother liquor and wash filtrate from an oxidizer purge stream produced in the synthesis of carboxylic acid, and routing at least a portion of the mother liquor and/or wash filtrate to an oxidation zone.

In a first embodiment of this invention, a process is provided. The process comprises:
(a) routing an oxidizer purge stream to a solids enrichment zone to form a purge slurry;
(b) subjecting said purge slurry to separation in a separation zone to produce a washed cake, mother liquor and a wash filtrate;
(c) routing at least a portion of said wash filtrate and at least a portion of said mother liquor to an oxidation zone.

In another embodiment of the invention, a process is provided. The process comprises:
(a) adding a precipitant agent to an oxidizer purge stream in a solids enrichment zone to form a purge slurry;
(b) subjecting said purge slurry to separation in a separation zone to produce a washed cake, mother liquor and a wash filtrate; and
(c) routing at least a portion of said wash filtrate or at least a portion of said mother liquor to an oxidation zone.

In another embodiment of the invention, a process is provided. The process comprises:
(a) cooling an oxidizer purge stream in a solids enrichment zone to form a purge slurry;
(b) subjecting said purge slurry to separation in a separation zone to produce a washed cake, mother liquor and a wash filtrate; and
(c) routing at least a portion of said wash filtrate or at least a portion of said mother liquor to an oxidation zone.

These objects, and other objects, will become more apparent to others with ordinary skill in the art after reading this disclosure.

### BRIEF DESCRIPTION OF THE DRAWINGS

Figure 1 illustrates a TPA manufacturing process where at least a portion of the combined wash filtrate **148** and/or mother liquor **147** is recycled back to the oxidation zone **400** via conduit **154.**

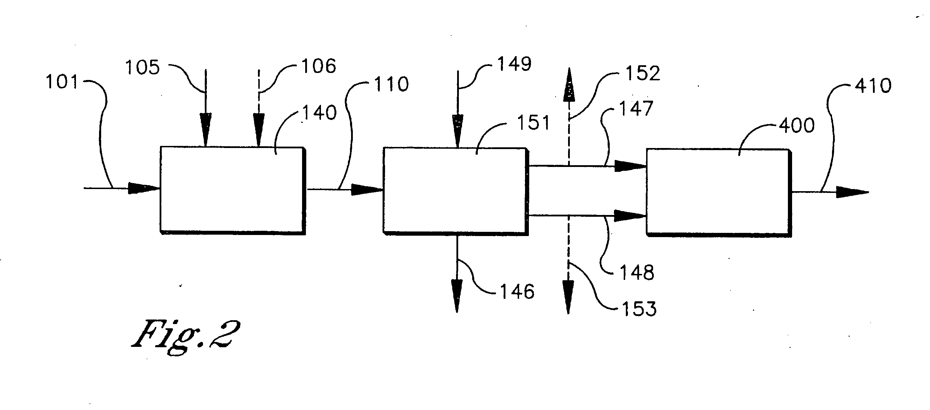

Figure 2 illustrates an embodiment of the invention of the process occurring in the separation zone **151** wherein a mother liquor **147** and a wash filtrate **148** are produced and then a portion of the mother liquor **147** and/or wash filtrate **148** are routed to an oxidation zone comprising at least one oxidation reactor.
Figure 3 illustrates another embodiment wherein the purge slurry **110** is routed to a separation zone **151** comprising a filtration zone **153,** and a washing zone **155,** and optionally, a drying zone **157.**
Figure 4 illustrates another embodiment of the invention where a rotary drum pressure filter is utilized as the process filtration device. In another embodiment of the invention, the rotary drum pressure filter comprises a filtration zone **153,** a wash zone **155,** optionally, a dewatering zone **157,** a discharge zone **164** and a cloth wash zone **162.**

### DESCRIPTION OF THE INVENTION

In one embodiment of this invention, a process to remove impurities and to recover a mother liquor **147** and a wash filtrate **148** from an oxidizer purge stream **101** is provided as shown in Figures 1 though 4. Common numbers within the Figures indicate the same process stream. The process comprises the following steps.

The oxidizer purge stream **101** is withdrawn from a carboxylic acid oxidative synthesis process. The oxidizer purge stream **101** serves as the feed stream to the present process. The oxidizer purge stream **101** comprises carboxylic acid, water, a solvent, metal catalyst and at least one impurity. The impurity comprises at least one selected from the group consisting of organic bromides, corrosion metals, p-xylene oxidation by-products, and impurities derived as a result of impurities in the p-xylene. The organic bromides may be used as promoters in the oxidation reaction. Examples of corrosion metals are iron and chromium compounds, which inhibit, reduce or entirely destroy the activity of the metal catalyst. Aside from the catalyst and promoter, the oxidizer mother liquor stream **415** also contains by-products and impurities. These by-products and impurities arise partially from minor impurities present in the p-xylene feed stream. Other impurities arise due to the incomplete oxidation of p-xylene resulting in partially oxidized products. Still other by-products result from competing side reactions in the oxidation of p-xylene to terephthalic acid.

Carboxylic acids include aromatic carboxylic acids produced via controlled oxidation of an organic substrate. Such aromatic carboxylic acids include compounds with at least one carboxylic acid group attached to a carbon atom that is part of an aromatic ring, preferably having at least 6 carbon atoms, even more preferably having only carbon atoms. Suitable examples of such aromatic rings include, benzene, biphenyl, terphenyl, naphthalene, and other carbon-based fused aromatic rings. Examples of suitable carboxylic acids include, terephthalic acid, benzoic acid, p-toluic acid, isophthalic acid, trimellitic acid, naphthalene dicarboxylic acid, 2,5-diphenyl-terephthalic acid and mixtures thereof.

Suitable solvents include, aliphatic mono- carboxylic acids, preferably containing 2 to 6 carbon atoms, or benzoic acid and mixtures thereof and mixtures of these compounds with water. Preferably, the solvent is acetic acid mixed with water, in a ratio of 5:1 to 25:1, preferably between 8:1 and 20:1. Throughout the specification, acetic acid will be referred to as the solvent. However, it should be appreciated that other suitable solvents, such as those disclosed previously, may also be utilized.

### Step (a) comprises routing an oxidizer purge stream 101 to a solids enrichment zone 140 to form a purge slurry 110.

In one embodiment of the invention, the solids enrichment zone **140** comprises a vessel suitable to allow cooling of the oxidizer purge stream **101** to produce the purge slurry **110.** There are no particular requirements for this vessel other than it allows enough residence time to cool the oxidizer purge stream **101** to the desired temperature to produce the purge slurry **110.** This can be accomplished by, for example, using an agitated vessel comprising cooling coils, or utilizing various heat exchangers known in the art. The solids enrichment zone **140** can comprise any device known in the art sufficient to cool the oxidizer purge stream **101** sufficiently to produce a purge slurry **110** wherein the oxidizer purge stream **101** has been cooled from at least 5°C to at least 90°C. The amount of cooling will depend on the amount of precipitation that is desired. For example, the cooling of the oxidizer purge stream **101** can be at least 5°C which is the difference in the temperature in Celsius between the oxidizer purge stream **101** and the purge slurry **110**. Another range could be at least 10°C, at least 15°C, at least 20°C, at least 25°C, at least 30°C, at least 35°C, at least 40°C, at least 45°C, at least 50°C, at least 55°C, at least 60°C, at least 65°C, at least 70°C, at least 75°C, at least 80°C, at least 85°C, and at least 90°C. In another embodiment of the invention, the oxidizer purge stream **101** is at a temperature of 80°C to 150°C. Another range is 80°C to 140°C and another range is 85°C to 100°C.

In another embodiment the oxidizer purge stream **101** is charged to a solids enrichment zone **140** to cause solids to precipitate. The precipitation can be accomplished by any means known in the art. For example, one method is the addition of a precipitant agent stream **105** to the oxidizer purge. The precipitant agent **105** is charged to the solids enrichment zone **140** and causes precipitation of solids from the oxidizer purge stream **101.** The precipitant agent **105** comprises any compound suitable to precipitate solids from the oxidizer purge stream **101.** Suitable compounds include, water, methanol, isopropanol, n- butanol, and isobutanol. Other precipitant agents **105** can be used are c1 to c6 alkyl acetates, such as n-propyl acetate, isopropyl acetate, isobutyl acetate, sec-butyl acetate, ethyl acetate, water, and n- butyl acetate.

In another embodiment of the invention for step (a) the oxidizer purge stream **101** can be concentrated by the removal of acetic acid. The concentration can be accomplished by any means known in the art. This can be accomplished by boiling away acetic acid to the extent that there is insufficient acetic acid solvent to keep the solute solids in solution. Another method of solvent removal is the use of a cross flow membrane system that allows acetic acid to cross the membrane as permeate resulting in a concentrate retentate.

In another embodiment of the invention for step (a) solids can be generated in the solids enrichment zone by any combination of cooling and precipitant agent addition.

In another embodiment of the invention the amount of solid in the solid(s) enrichment process can result in the purge slurry **110** having at least 1 % by weight solids to at least 30 % by weight solids. Another range is at least 1 % by weight solids to at least 25 % by weight solids, and another range is at least 1 % by weight solids to at least 20 % by weight solids. The solids enrichment can be accomplished by the cooling process described previously or in another embodiment of the invention, the purge slurry **110** can be produced from the oxidizer purge stream **101** though the use of evaporation of the solvent. Fox example, in another embodiment of the invention the solids enrich zone comprises an evaporator that could be used to evaporate the solvent. The evaporator can be an evaporator such as the one used in U. S patent #4,939,297, or in U. S patent applications 10/455,016, 10/455,018 and 10/874,419.

In another embodiment of the invention, any means known in the art can be used so long as the desired content of solids in the purge slurry **110** is obtained.

Step (b) comprises separating the purge slurry **110** in a separation zone **151** to form a filter cake **154** and a mother liquor **147;** and washing the filter cake **154** with a wash feed **149** in the separation zone **151** to form a washed cake **146** and a wash filtrate **148;** and optionally dewatering the washed cake **146** in the separation zone **151** to form a dewatered cake **159;** wherein the separation zone **151** comprises at least one pressure filtration device;

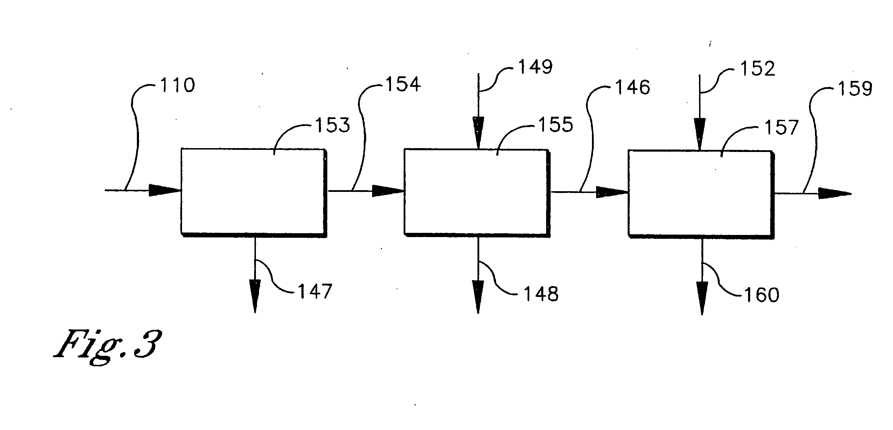

In another embodiment of the invention, the purge slurry **110** is introduced in the separation zone **151** wherein the separation zone comprises a filtration zone **153,** a washing zone **155,** and optionally a drying zone **157** as shown in Figure 3. The filtration zone **153** comprises a filter cell, or a series of filter cells, physically situated to permit a filter cake **154** to develop a distribution across the area of the filter cell to hinder or prevent the channeling of wash feed **149** through the filter cake **154.**

A filter cake **154** of at least 6.35 mm (0.25 inch) in depth to 203.20 mm (8 inches) in depth, preferably at least 12.70 mm (0.5 inch) in depth, more preferably at least 25.40 mm (1 inch) in depth, and even more preferably 50.80 mm (2 inch) to 101.60 mm (4 inches) in depth is distributed over the area of the filter cell. The washed cake, **146,** can be recovered or further treated, recycled and/or sent to waste treatment facilities.

Upon obtaining a suitable or preferred height of filter cake **154,** 12.70 mm (0.5 inch) to 101.60 mm (4 inch), the filter cake **154** leaves the filtration zone **153** which comprises a filter or series of filters and enters a washing zone **155** where the filter cake **154** is contacted with a wash feed **149.** There is sufficient pressure across the filter cake **154** to allow a reservoir or buildup of the wash feed **149** over the filter cake **154** to a suitable depth, preferably to a minimum depth of 6.35 mm (0.25 inch). A pressure gradient of at least 3447 Pa (0.5 psi), preferably from 34474 Pa (5 psi) to 448159 Pa (65 psi). across the filter cake **154** and the reservoir of wash feed **149** can be applied to displace any solute in the filter cake **154** with wash feed **149.**

A filter cake **154** depth of at least 12.70 mm (0.5 inch) is suitable to obtain a filter cake **154** of sufficient compactness to furnish a wash vehicle, i.e. the filter cake **154,** from which a wash filtrate **148** containing a solute from the filter cake **154** can be removed efficiently by displacement washing. If the filter cake depth **154** is less than 6.35 mm (0.25 inch), channeling of wash feed **149** in the filter cake **154** can occur resulting in non-uniform washing of the filter cake **154.**

Because of the loss of efficiency in displacement washing of the filter cake **154,** a minimum filter cake **154** depth of at least 6.35 mm (0.25 inch), of purified terephthalic acid is preferred.

A minimum liquid height above the filter cake **154** surface is required to ensure that displacement washing occurs. This height must be sufficient to ensure that the filter cake **154** surface is completely covered with wash feed **149.** If the filter cake **154** surface is not covered with wash feed **149,** bypassing of the wash feed 149 can occur without adequate displacement of the solute in the filter cake **154.** Because of irregularities in the filter cake **154** surface, a minimum liquid height of 6.35 mm (0.25 inch) is preferred above the filter cake **154** surface.

It has been found that displacement of the solute from the filter cake **154** using the wash feed **149** at high pressure permits an efficient separation of catalyst metals from the filter cake **154.** Another benefit of the high pressure is the reduction of wash feed **149** required to recover cobalt as shown in the examples.

Utilization of added stages in the separation zone **151** can decrease the amount of wash feed **149** required to reduce the total amount of metal catalyst retained in the filter cake **154.** It is convenient therefore that a suitable number of stages of positive displacement washing be used to minimize total wash feed **149** used in displacement washing to reduce need for downstream waste treatment facilities.

It is understood that multiple stages of the displacement washing procedure can replace a single stage displacement washing procedure wherein the quantity of wash feed **149** is sufficient to obtain at least 80 % recovery of the metal catalyst from the purge slurry **110** to the mother liquor **147** and the wash filtrate **148.** Additionally, a procedure utilizing multiple stages of counter-current washing can be useful if reduction of the amount of wash feed **149** is determined to be advantageous.

In the process of the instant invention, a purge slurry **110** is introduced into one or more of a series of filter cells physically situated to permit a filter cake **154** of requisite thickness to develop.

Upon obtaining a minimum height of filter cake **154,** 6.35 mm (0.25 inch) to 101.60 mm (4 inch), the filter cake **154** leaves the filter or series of filters and enters a washing zone **155** where the filter cake **154** is washed with a wash feed **149.** Pressure can then be applied to the wash feed **149** to displace the solute (i.e. the liquid and any dissolved compounds such as metal catalyst in the filter cake) of the filter cake **154.** Upon displacement of the solute with the wash feed, the filter cake **154** can be discharged from the filtration zone **155** by any suitable means and the cycle repeated. In an embodiment of the invention the ratio of wash feed **149** to filter cake **154** discharge is within the range of from 1:20 to 20:1 to reduce the level of metal catalyst in the filter cake by greater than 95 %.

Equipment for performing the requisite washing cycle can comprise a series of filter cells maintained in a suitable position to permit a wash feed **149** flood to develop over the filter cells. In one embodiment of the invention, suitable equipment can comprise a rotary drum pressure filter with multiple filter cells, fitted with a means for discharging washed cake **146** from the filter cells. The filter cake **154** can be washed for as many times as required to develop a minimum concentration of metal catalyst in the washed cake **146** before discharging the washed cake **146** from the rotary drum filter.

A suitable pressure filter which can be adapted to the requirements of the instant invented process is a BHS-FEST™ rotary drum pressure filter, BHS-WERK, Sonthofen, D-8972, Sonthofen, West Germany, although other pressure filters which can accomplish the required operation can be used. Examples of other devices that can used in the solid-liquid separation zone **151** include, pressure belt filters, filter presses, centrifuges, pressure leaf filters, and cross-flow filters. The pressure filter can be operated at a temperature and pressure sufficient to obtain at least 80 % recovery of the metal catalyst from the solute of the mother liquor **147.** Preferably, the pressure filter can be operated at a temperature of 25°C to 160°C and a pressure of 1 atmospheres to 50 atmospheres.

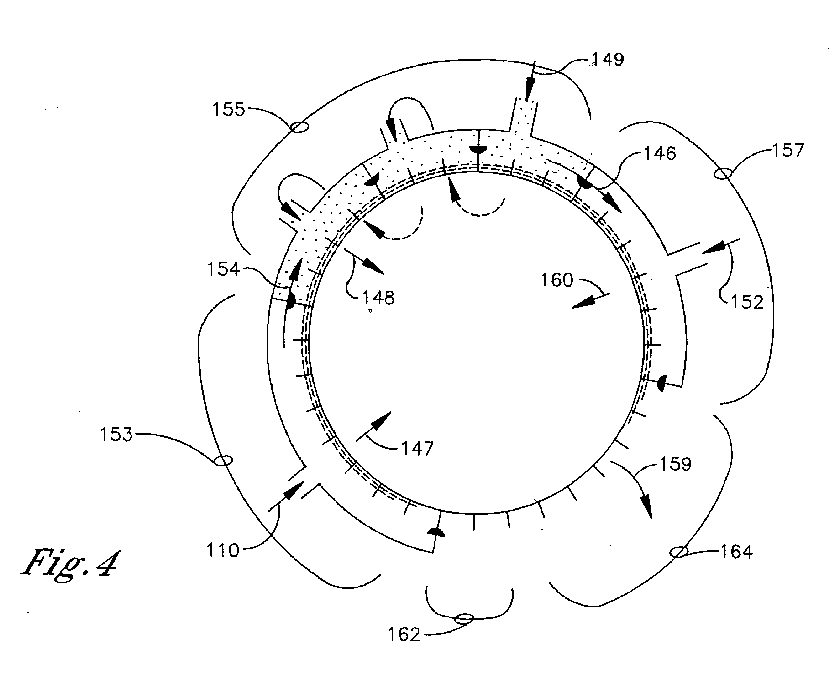

In the operation of the BHS-FEST™ filter as shown in Figure 4, a rotary drum contains a series of filter cells located on the periphery of the rotating drum. As the drum rotates, the filter cells receive a purge slurry **110** and a filter cake **154** builds to a requisite depth. The mother liquor **147** is produced by filtration of the purge slurry **110.** Upon rotation of the drum, the filter cake **154** enters a washing zone **155** where reservoir of wash feed **149** is built up over the filter cake **154** to a required depth. The applied pressure to the wash feed reservoir forces the water through the filter cake **154** to displace the solute (with dissolved metal catalyst) retained in the purge slurry **110** to produce a washed cake **146.** Upon further rotation of the drum, the wash cycle can be repeated at least three more times if necessary in a counter current fashion, after which the system pressure is released with attendant temperature decrease to an ambient conditions. Optionally, the washed cake **146** can be dewatered in a dewatering zone **157** with a vapor via conduit **152** to produce a dewatered cake **159** and a humid vapor **160.** The resultant dewatered cake **159** can then be discharged from the drum by any conventional means.

Figure 4 illustrates an embodiment of the invention where a rotary pressure drum filter is utilized as the process filtration device. The rotary drum pressure filter comprises a filtration zone **153,** a wash zone **155,** optionally, a dewatering zone **157,** a discharge zone **164** and a cloth wash zone **162.** The cloth wash zone **164** shown in figure 4 is an embodiment of the invention where the rotary pressure drum filter comprises a cloth wash zone **162** where the filters are washed after discharge of the dewatered cake **159.**

The wash filtrate **148** is produced by displacement washing the filter cake with the wash feed **149.** The filter cake **154** within the separation zone **151** undergoes extraction of metal catalyst by introduction of the wash feed **149** to form the wash filtrate **148** wherein in an embodiment of the invention at least 80 % of the metal catalyst is recovered in the wash filtrate and the mother liquor **147.** In an embodiment of the invention, at least 90 % of the metal catalyst is recovered in the wash filtrate **148** and the mother liquor **147.** The mother liquor **147** and the wash filtrate **148** can optionally be combined before exiting the solid-liquid separation zone **151.**

The wash feed **149** comprises water and optionally an additional oxidation solvent.

Perhaps most surprisingly, is that by utilizing water as a wash feed **149** at temperatures in the range of 20°C to 70°C preferably 30°C to 50 degrees C, sufficient corrosion metal is retained in the dewatered cake **159** wherein the need for corrosion metal removal by other means is eliminated. The dewatered cake **159** which represents solids stripped of metal catalyst can be disposed from the system.

It should be noted that one embodiment of the invention utilizing a rotary pressure drum filter the separation zone **151** can comprises any device known in the art sufficient to produce a mother liquor **147** and wash filtrate **148** from the purge slurry **110.** For example such devices can include a centrifuge, decanter centrifuge, stack disk centrifuge, pressure filters such as candle filters, leaf filters, filter press and the like.

### Step (c) comprises routing at least a portion of the wash filtrate and/or at least a portion of the mother liquor to an oxidation zone.

In an embodiment of the invention at least a portion of the mother liquor **147** and/or at least a portion of the wash filtrate **148** in step (e) can be recycled back to the oxidation zone **400** comprising at least one oxidation reactor in a terephthalic acid process, such as, the process described in figure 1. At least a portion can be any amount greater than 1 % by weight. In another range, at least a portion can be any amount greater than 25 % by weight. In another range, at least a portion can be any amount greater than 50 % by weight. In another range, at least a portion can be any amount greater than 75 % by weight. In another range, at least a portion can be 100 % by weight. Conduits **152** and **153** represent the portion, if any, of mother liquor **147** and wash filtrate **148** respectfully that is not recycled back to the oxidation zone **400.** These streams can be used in other processes or disposed of through incineration or any other means known in the art.

## Claims

1. A process for removal of impurities from an oxidizer purge stream comprising:
(a) routing an oxidizer purge stream obtained from a process for producing carboxylic acids via catalyzed oxidation, wherein the oxidizer purge stream comprises carboxylic acid, water, solvent, catalyst and at least one impurity, to a solids enrichment zone to form a purge slurry, wherein the amount of solid generated in the solids enrichment zone can result in the purge slurry having at least 1% by weight solids to at least 30% by weight solids;
(b) subjecting said purge slurry to separation in a separation zone to produce a washed cake, mother liquor and a wash filtrate, said separation zone comprises at least one pressure filtration device;
(c) routing at least a portion of said wash filtrate and at least a portion of said mother liquor to an oxidation zone of said process for producing carboxylic acids via catalyzed oxidation.

2. The process according to claim 1 wherein said pressure filtration device comprises at least one filter cell and wherein at least one filter cell accumulates at least 6.35 mm (0.25 inch) in depth of said filter cake.

3. The process according to claim 2 wherein said wash feed forms a reservoir over said filter cake which is at least 6.35 mm (0.25 inch) in depth.

4. The process according to claim 3 wherein said pressure filtration device operates at a temperature between 25°C to 160°C.

5. The process according to claim 4 wherein said pressure filtration device is operated at a pressure of 101325 Pa (1 atmosphere) to 5066250 Pa (50 atmosphere).

6. The process according to claim 5 wherein said dewatering results in said dewatered cake having a moisture content from 10% to 50%.

7. The process according to claim 6 wherein said pressure filtration device is a rotary pressure drum filter.

8. The process according to claim 1 or 7 wherein said washing is counter current.

9. The process according to claim 1 or 7 wherein said pressure filtration device is operated at a pressure of 101325 Pa (1 atmosphere) to 5066250 Pa (50 atmosphere).

10. The process for removal of impurities from an oxidizer purge stream as claims in claim 1, wherein a precipitant agent is added to the oxidizer purge stream in the solids enrichment zone.

11. The process according to claim 10 wherein said pressure filtration device comprises at least one filter cell and wherein at least one filter cell accumulates at least 6.35 mm (0.25 inch) in depth of said filter cake.

12. The process according to claim 11 wherein said wash feed forms a reservoir over said filter cake which is at least 6.35 mm (0.25 inch) in depth.

13. The process according to claim 12 wherein said pressure filtration device operates at a temperature between 25°C to 160°C.

14. The process according to claim 10 wherein step (c) comprises routing at least a 10 percent by weight of said wash filtrate or at least a portion of said mother liquor to an oxidation zone.

15. The process according to claim 10 wherein step (c) comprises routing at least a 50 percent by weight of said wash filtrate or at least a portion of said mother liquor to an oxidation zone.

16. The process according to claim 10 or 15 wherein said pressure filtration device is a rotary pressure drum filter.

17. The process according to claim 10 or 15 wherein said washing is counter current.

18. The process according to claim 10 wherein said pressure filtration device is operated at a pressure of 101325 Pa (1 atmosphere) to 5066250 Pa (50 atmosphere).

19. The process according to claim 10 wherein at least 25 percent by weight of the wash filtrate and 25 percent by weight of the mother liquor is routed to an oxidation zone.

20. The process according to claim 19 wherein said separation zone comprises at least one pressure filtration device.

21. The process according to claim 20 wherein said pressure filtration device comprises at least one filter cell and wherein at least one filter cell accumulates at least 6.35 mm (0.25 inch) in depth of said filter cake.

22. The process according to claim 21 wherein said wash feed forms a reservoir over said filter cake which is at least 6.35 mm (0.25 inch) in depth.

23. The process according to claim 22 wherein said pressure filtration device operates at a temperature between 25°C to 160°C.

24. The process according to claim 23 wherein said pressure filtration device is operated at a pressure of 101325 Pa (1 atmosphere) to 5066250 Pa (50 atmosphere).

25. The process according to claim 24 wherein step (c) comprises routing at least a 50 percent by weight of said wash filtrate and 50 percent by weight of said mother liquor to an oxidation zone.

26. The process according to claim 19 or 25 wherein said pressure filtration device is a rotary pressure drum filter.

27. The process according to claim 19 or 25 wherein said washing is counter current.

28. The process according to claim 19 wherein said pressure filtration device is operated at a pressure of 101325 Pa (1 atmosphere) to 5066250 Pa (50 atmosphere).

29. The process for removal of impurities from an oxidizer purge stream as claimed in claim 1, wherein cooling the oxidizer purge stream is cooled in the solids enrichment zone.

30. The process according to claim 29 wherein said pressure filtration device comprises at least one filter cell and wherein at least one filter cell accumulates at least 6.35 mm (0.25 inch) in depth of said filter cake.

31. The process according to claim 30 wherein said wash feed forms a reservoir over said filter cake which is at least 6.35 mm (0.25 inch) in depth.

32. The process according to claim 31 wherein said pressure filtration device operates at a temperature between 25°C to 160°C.

33. The process according to claim 29 wherein step (c) comprises routing at least a 10 percent by weight of said wash filtrate or at least a portion of said mother liquor to an oxidation zone.

34. The process according to claim 29 wherein step (c) comprises routing at least a 50 percent by weight of said wash filtrate or at least a portion of said mother liquor to an oxidation zone.

35. The process according to claim 29 or 34 wherein said pressure filtration device is a rotary pressure drum filter.

36. The process according to claim 29 or 34 wherein said washing is counter current.

37. The process according to claim 36 wherein said pressure filtration device is operated at a pressure of 101325 Pa (1 atmosphere) to 5066250 Pa (50 atmosphere).

38. The process according to claim 37 wherein at least 25 percent by weight of the wash filtrate and 25 percent by weight of the mother liquor is routed to an oxidation zone.

39. The process according to claim 38 wherein said separation zone comprises a pressure filtration device that is operated at a pressure of 101325 Pa (1 atmosphere) to 5066250 Pa (50 atmosphere).

40. The process according to claim 39 wherein said pressure filtration device comprises at least one filter cell and wherein at least one filter cell accumulates at least 6.35 mm (0.25 inch) in depth of said filter cake.

41. The process according to claim 40 wherein said wash feed forms a reservoir over said filter cake which is at least 6.35 mm (0.25 inch) in depth.

42. The process according to claim 41 wherein said pressure filtration device operates at a temperature between 25°C to 160°C.

43. The process according to claim 42 wherein said pressure filtration device is operated at a pressure of 101325 Pa (1 atmosphere) to 5066250 Pa (50 atmosphere).

44. The process according to claim 43 wherein step (c) comprises routing at least a 50 percent by weight of said wash filtrate and 50 percent by weight of said mother liquor to an oxidation zone.

45. The process according to claim 38 or 44 wherein said pressure filtration device is a rotary pressure drum filter.

46. The process according to claim 38 or 44 wherein said washing is counter current.

47. The process according to claim 38 wherein said pressure filtration device is operated at a pressure of 101325 Pa (1 atmosphere) to 5066250 Pa (50 atmosphere).

## Patentansprüche

1. Verfahren zum Entfernen von Verunreinigungen aus einem Oxidationsmittel-Spülstrom umfassend:
(a) Leiten eines Oxidationsmittel-Spülstroms, der aus einem Verfahren zur Herstellung von Carbonsäuren mittels katalysierter Oxidation erhalten wird, wobei der Oxidationsmittel-Spülstrom Carbonsäure, Wasser, Lösungsmittel, Katalysator und mindestens eine Verunreinigung umfasst, in eine Anreicherungszone für Feststoffe, um eine Spülaufschlämmung zu bilden, wobei die Menge an Feststoff, die in der Anreicherungszone für Feststoffe erzeugt wird, in einer Spülaufschlämmung resultieren kann, die mindestens 1 Gew.-% Feststoffe bis mindestens 30 Gew.-% Feststoffe aufweist;
(b) Unterwerfen besagter Spülaufschlämmung der Trennung in einer Trennzone, um einen gewaschenen Kuchen, Mutterlauge und ein Waschfiltrat herzustellen, wobei besagte Trennzone mindestens eine Druckfiltrationsvorrichtung aufweist;
(c) Leiten mindestens eines Teils des besagten Waschfiltrats und mindestens eines Teils der besagten Mutterlauge in eine Oxidationszone des besagten Verfahrens zur Herstellung von Carbonsäuren mittels katalysierter Oxidation.

2. Verfahren nach Anspruch 1, wobei besagte Druckfiltrationsvorrichtung mindestens eine Filterzelle umfasst, und wobei mindestens eine Filterzelle besagten Filterkuchen in einer Dicke von mindestens 6,35 mm (0,25 Inch) anreichert.

3. Verfahren nach Anspruch 2, wobei besagte Waschzufuhr ein Reservoir über besagtem Filterkuchen bildet, welches mindestens 6,35 mm (0,25 Inch) dick ist.

4. Verfahren nach Anspruch 3, wobei besagte Druckfiltrationsvorrichtung bei einer Temperatur zwischen 25 °C bis 160 °C arbeitet.

5. Verfahren nach Anspruch 4, wobei besagte Druckfiltrationsvorrichtung bei einem Druck von 101.325 Pa (1 Atmosphäre) bis 5.066.250 Pa (50 Atmosphären) betrieben wird.

6. Verfahren nach Anspruch 5, wobei besagtes Entwässern in besagtem entwässerten Kuchen resultiert, der einen Feuchtigkeitsgehalt von 10 % bis 50 % aufweist.

7. Verfahren nach Anspruch 6, wobei besagte Druckfiltrationsvorrichtung ein rotierender Trommeldruckfilter ist.

8. Verfahren nach Anspruch 1 oder 7, wobei besagtes Waschen im Gegenstrom verläuft.

9. Verfahren nach Anspruch 1 oder 7, wobei besagte Druckfiltrationsvorrichtung bei einem Druck von 101.325 Pa (1 Atmosphäre) bis 5.066.250 PA (50 Atmosphären) betrieben wird.

10. Verfahren zum Entfernen von Verunreinigungen aus einem Oxidationsmittel-Spülstrom wie in Anspruch 1 beansprucht, wobei ein Niederschlagsmittel dem Oxidationsmittel-Spülstrom in der Anreicherungszone für Feststoffe hinzugefügt wird.

11. Verfahren nach Anspruch 10, wobei besagte Druckfiltrationsvorrichtung mindestens eine Filterzelle umfasst, und wobei mindestens eine Filterzelle besagten Filterkuchen in einer Dicke von mindestens 6,35 mm (0,25 Inch) anreichert.

12. Verfahren nach Anspruch 11, wobei besagte Waschzufuhr ein Reservoir über besagtem Filterkuchen bildet, welches mindestens 6,35 mm (0,25 Inch) dick ist.

13. Verfahren nach Anspruch 12, wobei besagte Druckfiltrationsvorrichtung bei einer Temperatur zwischen 25 °C bis 160 °C arbeitet.

14. Verfahren nach Anspruch 10, wobei besagter Schritt (c) das Leiten von mindestens 10 Gew.-% des besagten Waschfiltrats oder mindestens eines Teils der besagten Mutterlauge in eine Oxidationszone umfasst.

15. Verfahren nach Anspruch 10, wobei Schritt (c) das Leiten von mindestens 50 Gew.-% des besagten Waschfiltrats oder mindestens eines Teils der besagten Mutterlauge in eine Oxidationszone umfasst.

16. Verfahren nach Anspruch 10 oder 15, wobei besagte Druckfiltrationsvorrichtung ein rotierender Trommeldruckfilter ist.

17. Verfahren nach Anspruch 10 oder 15, wobei besagtes Waschen im Gegenstrom verläuft.

18. Verfahren nach Anspruch 10, wobei besagte Druckfiltrationsvorrichtung bei einem Druck von 101.325 Pa (1 Atmosphäre) bis 5.066.250 Pa (50 Atmosphären) betrieben wird.

19. Verfahren nach Anspruch 10, wobei mindestens 25 Gew.-% des Waschfiltrats und 25 Gew.-% der Mutterlauge in eine Oxidationszone geleitet werden.

20. Verfahren nach Anspruch 19, wobei besagte Trennzone mindestens eine Druckfiltrationsvorrichtung umfasst.

21. Verfahren nach Anspruch 20, wobei besagte Druckfiltrationsvorrichtung mindestens eine Filterzelle umfasst, und wobei mindestens eine Filterzelle besagten Filterkuchen in mindestens einer Dicke von 6,35 mm (0,25 Inch) anreichert.

22. Verfahren nach Anspruch 21, wobei besagte Waschzufuhr ein Reservoir über besagtem Filterkuchen bildet, welches mindestens 6,35 mm (0,25 Inch) dick ist.

23. Verfahren nach Anspruch 22, wobei besagte Druckfiltrationsvorrichtung bei einer Temperatur zwischen 25 °C bis 160 °C arbeitet.

24. Verfahren nach Anspruch 23, wobei besagte Druckfiltrationsvorrichtung bei einem Druck von 101.325 Pa (1 Atmosphäre) bis 5.066.250 Pa (50 Atmosphären) betrieben wird.

25. Verfahren nach Anspruch 24, wobei Schritt (c) das Leiten von mindestens 50 Gew.-% des besagten Waschfiltrats und 50 Gew.-% der besagten Mutterlauge in eine Oxidationszone umfasst.

26. Verfahren nach Anspruch 19 oder 25, wobei besagte Druckfiltrationsvorrichtung ein rotierender Trommeldruckfilter ist.

27. Verfahren nach Anspruch 19 oder 25, wobei besagtes Waschen im Gegenstrom verläuft.

28. Verfahren nach Anspruch 19, wobei besagte Druckfiltrationsvorrichtung bei einem Druck von 101.325 Pa (1 Atmosphäre) bis 5.066.250 Pa (50 Atmosphären) betrieben wird.

29. Verfahren zum Entfernen von Verunreinigungen aus einem Oxidationsmittel-Spülstrom wie in Anspruch 1 beansprucht, wobei das Kühlen des Oxidationsmittel-Spülstroms in der Anreicherungszone für Feststoffe erfolgt.

30. Verfahren nach Anspruch 29, wobei besagte Druckfiltrationsvorrichtung mindestens eine Filterzelle umfasst, und wobei mindestens eine Filterzelle besagten Filterkuchen in einer Dicke von mindestens 6,35 mm (0,25 Inch) anreichert.

31. Verfahren nach Anspruch 30, wobei besagte Waschzufuhr ein Reservoir über besagtem Filterkuchen bildet, welches mindestens 6,35 mm (0,25 Inch) dick ist.

32. Verfahren nach Anspruch 31, wobei besagte Druckfiltrationsvorrichtung bei einer Temperatur zwischen 25 °C bis 160 °C arbeitet.

33. Verfahren nach Anspruch 29, wobei Schritt (c) das Leiten von mindestens 10 Gew.-% des besagten Waschfiltrats oder mindestens eines Teils der besagten Mutterlauge in eine Oxidationszone umfasst.

34. Verfahren nach Anspruch 29, wobei Schritt (c) das Leiten von mindestens 50 Gew.-% des besagten Waschfiltrats oder mindestens eines Teils der besagten Mutterlauge in eine Oxidationszone umfasst.

35. Verfahren nach Anspruch 29 oder 34, wobei besagte Druckfiltrationsvorrichtung ein rotierender Trommeldruckfilter ist.

36. Verfahren nach Anspruch 29 oder 34, wobei besagtes Waschen im Gegenstrom verläuft.

37. Verfahren nach Anspruch 36, wobei besagte Druckfiltrationsvorrichtung bei einem Druck von 101.325 Pa (1 Atomsphäre) bis 5.066.250 Pa (50 Atmosphären) betrieben wird.

38. Verfahren nach Anspruch 37, wobei mindestens 25 Gew.-% des Waschfiltrats und 25 Gew.-% der Mutterlauge in eine Oxidationszone geleitet werden.

39. Verfahren nach Anspruch 38, wobei besagte Trennzone eine Druckfiltrationsvorrichtung umfasst, die bei einem Druck von 101.325 Pa (1 Atmosphäre) bis 5.066.250 Pa (50 Atmosphären) betrieben wird.

40. Verfahren nach Anspruch 39, wobei besagte Druckfiltrationsvorrichtung mindestens eine Filterzelle umfasst, und wobei mindestens eine Filterzelle besagten Filterkuchen in einer Dicke von mindestens 6,35 mm (0,25 Inch) anreichert.

41. Verfahren nach Anspruch 40, wobei besagte Waschzufuhr ein Reservoir über besagtem Filterkuchen bildet, welches mindestens 6,35 mm (0,25 Inch) dick ist.

42. Verfahren nach Anspruch 41, wobei besagte Druckfiltrationsvorrichtung bei einer Temperatur zwischen 25 °C bis 160 °C arbeitet.

43. Verfahren nach Anspruch 42, wobei besagte Druckfiltrationsvorrichtung bei einem Druck von 101.325 Pa (1 Atmosphäre) bis 5.066.250 Pa (50 Atomsphären) betrieben wird.

44. Verfahren nach Anspruch 43, wobei Schritt (c) das Leiten von mindestens 50 Gew.-% des besagten Waschfiltrats und 50 Gew.-% der besagten Mutterlauge in eine Oxidationszone umfasst.

45. Verfahren nach Anspruch 38 oder 44, wobei besagte Druckfiltrationsvorrichtung ein rotierender Trommeldruckfilter ist.

46. Verfahren nach Anspruch 38 oder 44, wobei besagtes Waschen im Gegenstrom verläuft.

47. Verfahren nach Anspruch 38, wobei besagte Druckfiltrationsvorrichtung bei einem Druck von 101.325 Pa (1 Atmosphäre) bis 5.066.250 Pa (50 Atmosphären) betrieben wird.

## Revendications

1. Procédé d'élimination d'impuretés d'un courant de purge oxydant comprenant :
(a) l'acheminement d'un courant de purge oxydant obtenu par un procédé de production d'acides carboxyliques via une oxydation catalysée, dans lequel le courant de purge oxydant comprend de l'acide carboxylique, de l'eau, un solvant, un catalyseur et au moins une impureté, vers une zone d'enrichissement en matières solides pour former une suspension épaisse de purge, dans lequel la quantité de matière solide générée dans la zone d'enrichissement en matières solides peut donner une suspension épaisse de purge comportant d'au moins 1 % en poids de matières solides à au moins 30 % en poids de matières solides ;
(b) la séparation de ladite suspension épaisse de purge dans une zone de séparation pour produire un gâteau lavé, une liqueur mère et un filtrat de lavage, ladite zone de séparation comprend au moins un dispositif de filtration sous pression ;
(c) l'acheminement d'au moins une partie dudit filtrat de lavage et d'au moins une partie de ladite liqueur mère vers une zone d'oxydation dudit procédé de production d'acides carboxyliques via une oxydation catalysée.

2. Procédé selon la revendication 1 dans lequel ledit dispositif de filtration sous pression comprend au moins une cellule filtrante et dans lequel au moins une cellule filtrante accumule au moins 6,35 mm (0,25 pouce) en profondeur dudit gâteau de filtration.

3. Procédé selon la revendication 2 dans lequel ladite charge de lavage forme un réservoir au-dessus dudit gâteau de filtration qui fait au moins 6,35 mm (0,25 pouce) de profondeur.

4. Procédé selon la revendication 3 dans lequel ledit dispositif de filtration sous pression fonctionne à une température située entre 25 °C et 160 °C.

5. Procédé selon la revendication 4 dans lequel ledit dispositif de filtration sous pression est utilisé à une pression de 101 325 Pa (1 atmosphère) à 5 066 250 Pa (50 atmosphères).

6. Procédé selon la revendication 5 dans lequel ladite déshydratation conduit à une teneur en humidité dudit gâteau déshydraté de 10 % à 50 %.

7. Procédé selon la revendication 6 dans lequel ledit dispositif de filtration sous pression est un filtre à tambour rotatif sous pression.

8. Procédé selon la revendication 1 ou 7 dans lequel ledit lavage est à contre-courant.

9. Procédé selon la revendication 1 ou 7 dans lequel ledit dispositif de filtration sous pression est utilisé à une pression de 101 325 Pa (1 atmosphère) à 5 066 250 Pa (50 atmosphères).

10. Procédé d'élimination d'impuretés d'un courant de purge oxydant selon la revendication 1, dans lequel un agent de précipitation est ajouté au courant de purge oxydant dans la zone d'enrichissement en matières solides.

11. Procédé selon la revendication 10 dans lequel ledit dispositif de filtration sous pression comprend au moins une cellule filtrante et dans lequel au moins une cellule filtrante accumule au moins 6,35 mm (0,25 pouce) en profondeur dudit gâteau de filtration.

12. Procédé selon la revendication 11 dans lequel ladite charge de lavage forme un réservoir au-dessus dudit gâteau de filtration qui fait au moins 6,35 mm (0,25 pouce) de profondeur.

13. Procédé selon la revendication 12 dans lequel ledit dispositif de filtration sous pression fonctionne à une température située entre 25 °C et 160 °C.

14. Procédé selon la revendication 10 dans lequel l'étape (c) comprend l'acheminement d'au moins 10 pour cent en poids dudit filtrat de lavage ou d'au moins une partie de ladite liqueur mère vers une zone d'oxydation.

15. Procédé selon la revendication 10 dans lequel l'étape (c) comprend l'acheminement d'au moins 50 pour cent en poids dudit filtrat de lavage ou d'au moins une partie de ladite liqueur mère vers une zone d'oxydation.

16. Procédé selon la revendication 10 ou 15 dans lequel ledit dispositif de filtration sous pression est un filtre à tambour rotatif sous pression.

17. Procédé selon la revendication 10 ou 15 dans lequel ledit lavage est à contre-courant.

18. Procédé selon la revendication 10 dans lequel ledit dispositif de filtration sous pression est utilisé à une pression de 101 325 Pa (1 atmosphère) à 5 066 250 Pa (50 atmosphères).

19. Procédé selon la revendication 10 dans lequel au moins 25 pour cent en poids du filtrat de lavage et 25 pour cent en poids de la liqueur mère sont acheminés vers une zone d'oxydation.

20. Procédé selon la revendication 19 dans lequel ladite zone de séparation comprend au moins un dispositif de filtration sous pression.

21. Procédé selon la revendication 20 dans lequel ledit dispositif de filtration sous pression comprend au moins une cellule filtrante et dans lequel au moins une cellule filtrante accumule au moins 6,35 mm (0,25 pouce) en profondeur dudit gâteau de filtration.

22. Procédé selon la revendication 21 dans lequel ladite charge de lavage forme un réservoir au-dessus dudit gâteau de filtration qui fait au moins 6,35 mm (0,25 pouce) de profondeur.

23. Procédé selon la revendication 22 dans lequel ledit dispositif de filtration sous pression fonctionne à une température située entre 25 °C et 160 °C.

24. Procédé selon la revendication 23 dans lequel ledit dispositif de filtration sous pression est utilisé à une pression de 101 325 Pa (1 atmosphère) à 5 066 250 Pa (50 atmosphères).

25. Procédé selon la revendication 24 dans lequel l'étape (c) comprend l'acheminement d'au moins 50 pour cent en poids dudit filtrat de lavage et 50 pour cent en poids de ladite liqueur mère vers une zone d'oxydation.

26. Procédé selon la revendication 19 ou 25 dans lequel ledit dispositif de filtration sous pression est un filtre à tambour rotatif sous pression.

27. Procédé selon la revendication 19 ou 25 dans lequel ledit lavage est à contre-courant.

28. Procédé selon la revendication 19 dans lequel ledit dispositif de filtration sous pression est utilisé à une pression de 101 325 Pa (1 atmosphère) à 5 066 250 Pa (50 atmosphères).

29. Procédé d'élimination d'impuretés d'un courant de purge oxydant selon la revendication 1, dans lequel le refroidissement du courant de purge oxydant est refroidi dans la zone d'enrichissement en matières solides.

30. Procédé selon la revendication 29 dans lequel ledit dispositif de filtration sous pression comprend au moins une cellule filtrante et dans lequel au moins une cellule filtrante accumule au moins 6,35 mm (0,25 pouce) en profondeur dudit gâteau de filtration.

31. Procédé selon la revendication 30 dans lequel ladite charge de lavage forme un réservoir au-dessus dudit gâteau de filtration qui fait au moins 6,35 mm (0,25 pouce) de profondeur.

32. Procédé selon la revendication 31 dans lequel ledit dispositif de filtration sous pression fonctionne à une température située entre 25 °C et 160 °C.

33. Procédé selon la revendication 29 dans lequel l'étape (c) comprend l'acheminement d'au moins 10 pour cent en poids dudit filtrat de lavage ou d'au moins une partie de ladite liqueur mère vers une zone d'oxydation.

34. Procédé selon la revendication 29 dans lequel l'étape (c) comprend l'acheminement d'au moins 50 pour cent en poids dudit filtrat de lavage ou d'au moins une partie de ladite liqueur mère vers une zone d'oxydation.

35. Procédé selon la revendication 29 ou 34 dans lequel ledit dispositif de filtration sous pression est un filtre à tambour rotatif sous pression.

36. Procédé selon la revendication 29 ou 34 dans lequel ledit lavage est à contre-courant.

37. Procédé selon la revendication 36 dans lequel ledit dispositif de filtration sous pression est utilisé à une pression de 101 325 Pa (1 atmosphère) à 5 066 250 Pa (50 atmosphères).

38. Procédé selon la revendication 37 dans lequel au moins 25 pour cent en poids du filtrat de lavage et 25 pour cent en poids de la liqueur mère sont acheminés vers une zone d'oxydation.

39. Procédé selon la revendication 38 dans lequel ladite zone de séparation comprend un dispositif de filtration sous pression qui est utilisé à une pression de 101 325 Pa (1 atmosphère) à 5 066 250 Pa (50 atmosphères).

40. Procédé selon la revendication 39 dans lequel ledit dispositif de filtration sous pression comprend au moins une cellule filtrante et dans lequel au moins une cellule filtrante accumule au moins 6,35 mm (0,25 pouce) en profondeur dudit gâteau de filtration.

41. Procédé selon la revendication 40 dans lequel ladite charge de lavage forme un réservoir au-dessus dudit gâteau de filtration qui fait au moins 6,35 mm (0,25 pouce) de profondeur.

42. Procédé selon la revendication 41 dans lequel ledit dispositif de filtration sous pression fonctionne à une température située entre 25 °C et 160 °C.

43. Procédé selon la revendication 42 dans lequel ledit dispositif de filtration sous pression est utilisé à une pression de 101 325 Pa (1 atmosphère) à 5 066 250 Pa (50 atmosphères).

44. Procédé selon la revendication 43 dans lequel l'étape (c) comprend l'acheminement d'au moins 50 pour cent en poids dudit filtrat de lavage et 50 pour cent en poids de ladite liqueur mère vers une zone d'oxydation.

45. Procédé selon la revendication 38 ou 44 dans lequel ledit dispositif de filtration sous pression est un filtre à tambour rotatif sous pression.

46. Procédé selon la revendication 38 ou 44 dans lequel ledit lavage est à contre-courant.

47. Procédé selon la revendication 38 dans lequel ledit dispositif de filtration sous pression est utilisé à une pression de 101 325 Pa (1 atmosphère) à 5 066 250 Pa (50 atmosphères).
